(19) Europäisches Patentamt
European Patent Office
Office européen des brevets

(11) Veröffentlichungsnummer: **0 147 867**
**A2**

(12) # EUROPÄISCHE PATENTANMELDUNG

(21) Anmeldenummer: 84116426.2

(22) Anmeldetag: 28.12.84

(51) Int. Cl.⁴: C 12 Q 1/48
C 12 Q 1/32, C 12 Q 1/26
C 12 Q 1/62, C 12 Q 1/28

(30) Priorität: 30.12.83 DE 3347636

(43) Veröffentlichungstag der Anmeldung:
10.07.85 Patentblatt 85/28

(84) Benannte Vertragsstaaten:
AT BE CH DE FR GB IT LI LU NL SE

(71) Anmelder: de Groot, Herbert, Dr.
Otto-Hahn-Strasse 123
D-4000 Düsseldorf(DE)

(72) Erfinder: de Groot, Herbert, Dr.
Otto-Hahn-Strasse 123
D-4000 Düsseldorf(DE)

(74) Vertreter: Hansen, Bernd, Dr.rer.nat. et al,
Hoffmann, Eitle & Partner Patentanwälte Arabellastrasse 4
D-8000 München 81(DE)

(54) **Verfahren zur enzymatischen Bestimmung von anorganischem Phosphat und Anwendungsverfahren.**

(57) Es wird ein Verfahren zur enzymatischen Bestimmung von anorganischem Phosphat und Anwendungsverfahren beschrieben, wonach anorganisches Phosphat in Gegenwart eines Nucleosids und biokatalytischer Mengen Nucleosidphosphorylase umgesetzt und entweder die gebildete Base oder Ribose-1-phosphat quantitativ bestimmt wird.

Weitere Anwendungsverfahren werden offenbart.

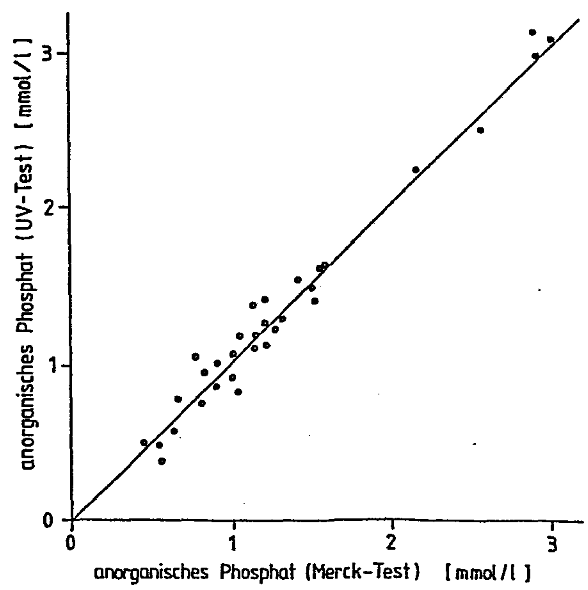


FIG. 1

EP 0 147 867 A2

Croydon Printing Company Ltd.

 0147867

Verfahren zur enzymatischen Bestimmung von anorganischem Phosphat und Anwendungsverfahren

Die Erfindung betrifft ein Verfahren zur enzymatischen Bestimmung von anorganischem Phosphat und Anwendungsverfahren.

In der klinischen Chemie wird anorganisches Phosphat in der Regel auf chemischem Wege als Phosphormolybdat bestimmt, welches durch geeignete Reduktionsmittel in Molybdänblau überführt wird. Hierzu wird z.B. auf "Test-Fibel", Firma Boehringer, Mannheim, 1983, und "Arbeitsanleitungen für die klinische Chemie", Firma Merck, Darmstadt, 1983, verwiesen. Diese Methode ist arbeitsaufwendig und mit zahlreichen weiteren Mängeln behaftet, wie dies z.B. von R. Richterich und J.P. Colombo in Klinische Chemie,. 4. Auflage, S. Karger, Basel, 1978, 403-411, beschrieben wird.

Neben dieser anorganisch-chemischen Methode sind auch enzymatische Methoden zur Phosphatbestimmung beschrieben worden. So wurde z.B. von D.W. Schulz, J.V. Passonneau und O.H. Lowry (1967) Anal. Biochem. 19, 300 - 314, eine Methode beschrieben, die sich die phosphorolytische Spaltung des Glycogens zu Nutze macht. Eine weitere Methode basiert auf der von der Glycerinaldehyd-3-phosphat-dehydrogenase katalysierten Reaktion der Glycolyse, wie dies von E.N. Fawaz und A. Tejirian (1972) in J. Clin. Chem. Clin. Biochem. 10, 215-219, und von R.K. Scopes, Anal. Biochem. 49 (1972), 88-94, beschrieben wird. Obgleich beide Methoden brauchbar sind und bei geeignetem Probematerial gute Ergebnisse liefern, konnten sie sich in der klinischen Routine bis zum heutigen Zeitpunkt nicht durchsetzen, was insbesondere auf methodenspezifische Mängel zurückzuführen ist. So ist z.B. die Glycogen-Phosphorylase-Methode nicht anwendbar bei Patientenseren mit einer erhöhten alpha-Amylase-Aktivität, und die Glycerinaldehyd-3-phosphat-dehydrogenase-Methode besitzt unter anderem den Nachteil, dass Phosphoester eingesetzt werden bzw. entstehen, die zum Teil wenig stabil sind und aus denen durch Phosphatasen Phosphat freigesetzt werden kann, wodurch das Ergebnis beeinflusst wird.

Aufgabe der vorliegenden Erfindung ist es, ein Verfahren zur enzymatichen Bestimmung des anorganischen Phosphats zur Verfügung zu stellen, welches im Gegensatz zur anorganisch-chemischen Bestimmung weniger arbeitsaufwendig, sehr empfindlich und hoch spezifisch ist. Im weiteren sollen die bisher auftretenden Mängel vermieden werden, so dass ein Routineeinsatz in der klinischen Chemie möglich ist.

0147867

Die vorstehende Aufgabe wird gemäss der Erfindung dadurch gelöst, dass anorganisches Phosphat in Gegenwart eines Nucleosids und biokatalytischer Mengen Nucleosid-phosphorylase umgesetzt und entweder die gebildete Base oder Ribose-1-phosphat quantitativ bestimmt wird.

Gemäss der Erfindung kann anstelle des Nucleosids auch das entsprechende Desoxyanaloge eingesetzt werden, wobei es zur Bildung von Desoxyribose-1-phosphat sowie der der Ausgangsverbindung entsprechenden Base kommt.

Die hier beschriebene, neu entwickelte enzymatische Bestimmung des anorganischen Phosphats basiert z.B. auf folgender, von Nucleosid-phosphorylase (EC 2.4.2.1) katalysierten Reaktion:

$$\text{Phosphat + Inosin} \xrightarrow{\text{Nucleosid-phosphorylase}} \text{Hypoxanthin + Ribose-1-phosphat}$$

Nucleosid-phosphorylase (EC 2.4.2.1) katalysiert neben Inosin auch die phosphorolytische Spaltung von Adenosin und Guanosin.

Um diese Reaktion messbar zu machen, bietet sich die Xanthinoxidase (EC 1.2.3.2) als Indikatorenzym an. Die Xanthinoxidase kann dabei als Oxidase oder als Dehydrogenase fungieren. Im folgenden werden diese beiden Funktionstypen der Xanthinoxidase-Aktivität beschrieben:

1. Die Xanthin-Oxidase arbeitet in ihrer physiologischen Oxidase-Funktion. Es wird $O_2$ reduziert und Hypoxanthin zu Harnsäure oxidiert. Die Reduktion des $O_2$ erfolgt unter anderem in Abhängigkeit vom pH-Wert, teilweise in einer Ein-Elektronen-Reduktion zu $O_2^-$ und teilweise in einer Zwei-Elektronen-Reduktion zu $H_2O_2$, wie dies von I. Fridovich (1970) J. Biol. Chem. 245, 4053-4057, beschrieben wird.

$$\text{Hypoxanthin} + 2O_2 + 2H_2O \xrightarrow{\text{Xanthinoxidase}} \text{Harnsäure} + 2H_2O_2 (4O_2^-)$$

Es bieten sich folgende Messmöglichkeiten an:

1.1 Fotometrische Bestimmung der gebildeten Harnsäure (z.B. bei 302 nm mit einem Filterfotometer der Firma Eppendorf).

1.2 Bestimmung des $O_2$-Verbrauches mit einer Sauerstoffelektrode.

1.3 Nachweis des gebildeten $O_2^-$ durch Reaktion mit Cytochrom $c^{3+}$ (siehe vorstehende Literatur von Fridovich):

$$\text{Cytochrom } c^{3+} + O_2^- \longrightarrow \text{Cytochrom } c^{2+} + O_2$$

Die Extinktion des reduzierten, rot gefärbten Cytochrom $c^{2+}$ kann fotometrisch bestimmt werden.

1.4 Nachweis des gebildeten $H_2O_2$

In diesem Fall kann die Empfindlichkeit dadurch gesteigert werden, dass durch Katalyse der Superoxiddismutase (EC 1.15.1.1) das gebildete $O_2^-$ ebenfalls in $H_2O_2$ überführt wird.

$$2O_2^- + 2H^+ \xrightarrow{\text{Superoxiddismutase}} H_2O_2 + O_2$$

1.4.1 Der Nachweis von $H_2O_2$ erfolgt durch folgende, von der Katalase (EC 1.11.1.6) und Aldehyd-dehydrogenase (EC 1.2.1.5) katalysierten Reaktionsabläufe in Anlehnung an R. Haeckel, J.Clin. Chem. Clin. Biochem. 14, (1976),101-107, wobei die Extinktion des gebildeten NADH fotometrisch bestimmt werden kann:

$$H_2O_2 + \text{Ethanol} \xrightarrow{\text{Katalase}} \text{Acetaldehyd} + 2H_2O$$

$$\text{Acetaldehyd} + NAD^+ + H_2O \xrightarrow{\text{Aldehyd-dehydrogenase}} \text{Acetat} + NADH + H^+$$

1.4.2 oder durch von der Peroxidase (EC 1.11.1.7) katalysierten Reaktion des $H_2O_2$ mit weiteren Substraten zu Farbstoffen, deren Extinktion fotometrisch bestimmt werden kann; eine brauchbare Reaktion ist beispielsweise die folgende, beschrieben von P. Fossati, L. Prencipe und G. Berti, Clin. Chem. 26, (1980) ,227-231:

$$2\ H_2O_2 + \text{Aminophenazon} + \text{3,5-Dichlor-benzolsulfonsäure} \xrightarrow{\text{Peroxidase}} \text{N-(4-Antipyryl)-3-chlor-5-sulfonat-p-benzochinonimin}$$

2. Die Xanthinoxidase fungiert als Dehydrogenase. Statt $O_2$ wird in dieser Funktion eine andere Substanz reduziert, beispielsweise ein Tetrazoliumsalz, wie von R. Fried, Anal. Biochem. 16, 1966, 427-432, beschrieben:

$$\text{Hypoxanthin} + 2\ \text{Tetrazoliumsalz} \xrightarrow{\text{Xanthinoxidase}} \text{Harnsäure} + 2\ \text{Formazan}$$

Die Extinktion des gebildeten Formazans lässt sich im sichtbaren Bereich fotometrisch bestimmen. Ein geeignetes Tetrazoliumsalz ist z.B. INT (2-(p-Jodophenyl)-3-(p-nitrophenyl)-5-phenyl-tetrazoliumchlorid). Die Reduktion des INT wird durch Sauerstoff nicht beeinflusst.

Neben diesen Reaktionswegen, die über die Xanthinoxidase als Hilfs- bzw. Indikatorenzym laufen, bietet sich auch die Möglichkeit, Ribose-1-phosphat quantitativ zu bestimmen, beispielsweise in Anlehnung an O.H. Lowry und J.V. Passonneau, J. Biol. Chem. 244, 1969, 920-926, nach dem folgenden Reaktionsweg:

$$\text{Ribose-1-phosphat} + \text{Glucose-1,6-diphosphat} \xrightarrow[\text{(EC 2.7.5.1)}]{\text{Phosphogluco-mutase}} \text{Ribose-1,5-diphosphat} + \text{Glycose-6-phosphat}$$

$$\text{Glucose-6-phosphat} + NADP^+ \xrightarrow[\text{(EC 1.1.1.49)}]{\text{Glucose-6-phosphat-dehydrogenase}} \text{6-Phospho-gluconolacton} + NADPH + H^+$$

Aufgrund der hohen Spezifität der Nucleosid-phosphorylase sind mit Hilfe des erfindungsgemässen Verfahrens keine Störungen durch Nebenreaktionen zu erwarten. Im Gegensatz zur Nucleosid-phosphorylase ist die Xanthinoxidase ein relativ unspezifisches Enzym. Sie kann ausser den physiologischen Substraten Hypoxanthin und Xanthin andere Purinderivate und auch Aldehyde umsetzen (siehe C. Walsh, 1979, Enzymatic Reaction Mechanism, Freeman and Company, San Francisco). Ausserdem wird ihre Aktivität gehemmt durch Arzneimittel, wie Allopurinol und 6-Mercaptopurin siehe C. Walsh, 1979, und H.R. Silberman und J.B. Wyngaarden, Biochim. Biophys. Acta 47, 1961, 178-180). Eine Störung durch möglicherweise im Serum vorhandene Substrate ist praktisch jedoch ohne Bedeutung, da

1. diese Substrate im Serum im Vergleich zum anorganischen Phosphat in einer wesentlich geringeren Konzentration vorliegen (in der Regel um den Faktor 10),

2. die nicht-physiologischen Substrate der Xanthinoxidase im Vergleich zu den physiologischen Substraten Hypoxanthin und Xanthin einen vielfach höheren $K_m$-Wert besitzen und mit einer wesentlich geringeren Geschwindigkeit ($V_{max}$) umgesetzt werden und darüber hinaus

3. die in der Klinik häufig eingesetzten dimethylierten Purinderivate Theophyllin, Theobromin und auch Coffein von der Xanthinoxidase nicht umgesetzt werden (siehe F. Bergmann und S. Dikstein, J. Biol. Chem. 223, 1956, 765-780). Eine mögliche Störung der Xanthinoxidase durch Hemmstoffe ist ebenfalls ohne praktische Konsequenz, zum einen aufgrund der hohen Verdünnung des eingesetzten Serums (1 : 200 bei den Farbtesten) und zum anderen aufgrund des Überschusses an Xanthinoxidase-Aktivität im Test.

Mögliche Störungen des NP-XOD-POD-Farbtestes (Methode 1.4.2) durch Peroxidase besitzen keine Relevanz, wie dies von P. Fossati, L. Prencipe und G. Berti, Clin. Chem. 26,(1980), 227-231, in einem anderen Zusammenhang untersucht wurde. Des weiteren besteht die Möglichkeit, dass Tetrazoliumsalze durch reduzierende Substanzen, wie z.B. Glutathion und Ascorbinsäure direkt reduziert werden können. Aufgrund der hohen Verdünnung des eingesetzten Serums ist jedoch auch dies ohne praktische Bedeutung.

Die Ergebnisse der praktischen Erprobung, die Analyse der Fehler- und Störmöglichkeiten und auch der Kostenvergleich zeigen, dass das erfindungsgemässe enzymatische Verfahren der Phosphatbestimmung der anorganisch-chemischen Methode deutlich überlegen ist und sich sehr gut für einen Routineeinsatz in der Klinik eignet. Dies gilt vor allem für die Farbteste. Sie kommen dem gegenwärtigen Trend in der klinischen Chemie entgegen, Teste einzusetzen, deren Endprodukt ein Absorptionsmaximum im

sichtbaren Bereich besitzt. Dies hat - in Verbindung mit der hohen Empfindlichkeit der Teste, welche eine hohe Verdünnung des Serums erlaubt - unter anderem den Vorteil, dass ein Probenleerwert entfallen kann. Von den beiden hier erprobten Farbtesten erscheint zur Zeit der NP-XOD-Farbtest (Methode 2) besonders vorteilhaft, da bei ihm auch auf das Mitführen eines Standards verzichtet werden kann.

Die erfindungsgemässe enzymatische Phosphatbestimmung erlaubt, neben dem beschriebenen Endwertverfahren, Phosphat auch auf kinetischer Basis zu bestimmen. Sie erfüllt somit sämtliche Anforderungen, die an eine automatengerechte Methode gestellt werden: wenige Pipettierschritte, kurze Analysenzeit und kein Probenleerwert.

Bisher wurde die enzymatische Phosphatbestimmung nur im Hinblick auf einen Routineeinsatz in der klinischen Chemie betrachtet. Sie eignet sich darüber hinaus natürlich auch für einen Einsatz in anderen Bereichen, so z .B. in der Forschung. Bei deren Anwendung in der Forschung sind die hohe Empfindlichkeit der enzymatischen Methode und die Tatsache, dass die Reaktionen im neutralen pH-Bereich ablaufen, von besonderem Vorteil. Letzteres erlaubt es, anorganisches Phosphat problemlos auch in Gegenwart von labilen Phosphoestern zu bestimmen. Dies ist bei der chemischen Methode mit Schwierigkeiten verbunden, da unter den dort gewählten Reaktionsbedingungen Phosphoester instabil sind.

0147867

Das erfindungsgemässe Verfahren der enzymatischen Bestimmung eignet sich nicht nur dazu, vorliegendes anorganisches Phosphat zu bestimmen, sondern es ist darüber hinaus möglich, die Aktivität Phosphat-freisetzender Enzyme mit geringem Arbeitsaufwand kontinuierlich und direkt zu bestimmen. Dies eröffnet ein grosser Feld möglicher weiterer Anwendungen. Durch Einsatz spezifischer Phosphatasen ist es schliesslich auch möglich, die Konzentration von Phosphoestern zu bestimmen.

Zusammenfassend besitzt die hier erfindungsgemässe enzymatische Methode der Phosphatbestimmung folgende Vorteile gegenüber den bisher bekannten Methoden:

1. grössere Spezifität,
2. höhere Empfindlichkeit,
3. kürzere Analysenzeit,
4. geringere Kosten aufgrund eines wesentlich geringeren Arbeitsaufwandes,
5. automatengerechtes Verfahren,
6. Analyse im neutralen pH-Bereich
7. Möglichkeit, die Aktivität Phosphat-freisetzender Emzyme kontinuierlich und direkt bestimmen zu können.

Die folgenden Versuche sollen das erfindugsgemässe Verfahren näher erläutern, ohne dieses zu beschränken. Die Vergleichsversuche zeigen die vorteilhaften Eigenschaften des erfindunsgemässen Verfahrens gegenüber einem bekannten, häufig angewendeten Verfahren zur Phosphatbestimmung.

<u>Vergleichsversuche:</u>

Von den vorstehend aufgeführten Reaktionskopplungen zur Phosphatbestimmung wurden drei ausgewählt und mit verschiedenen Patientenseren praktisch erprobt. Die Ergebnisse wurden mit denen verglichen, die mit der im Klinik-Labor üblichen anorganisch-chemischen Methode der Firma Merck ohne Enteiweissung (siehe Arbeitsanleitungen für die klinische Chemie, Firma Merck, Darmstadt, 1983) erhalten wurden.

Die eingesetzten Substrate sind in der Regel praktisch frei von anorganischem Phosphat. Die käuflichen Enzyme (z.B. von den Firmen Sigma, München, oder Boehringer, Mannheim) müssen hingegen von Phosphat befreit werden. Dies kann beispielsweise durch Dialyse gegen KCl/Tris-HCl-Puffer (104 mM/50 mM, pH 7,4) geschehen.

<u>Beispiel 1</u>

<u>NP-XOD-UV-Test</u> <u>(Methode 1.1)</u>

Die Bestimmung erfolgte nach folgendem Schema:

$$\text{Phosphat} + \text{Inosin} \xrightarrow[\text{(NP)}]{\text{Nucleosid-phosphorylase}} \text{Ribose-1-phosphat} + \text{Hypoxanthin}$$

$$\text{Hpyoxanthin} + 2O_2 + 2H_2O \xrightarrow[\text{(XOD)}]{\text{Xanthinoxidase}} \text{Harnsäure} + 2H_2O_2 (4O_2^-)$$

Es wird die folgende Reaktionslösung hergestellt:

| | |
|---|---|
| KCl/Tris-HCl-Puffer, pH 7,4 | 104 mM/50 mM |
| Ethlyendiamintetraacetat | 1 mM |
| Inosin | 0,2 mM |
| Nucleosid-phosphorylase | 500 U/l |
| Xanthinoxidase | 20 U/l |

Die Bestimmung erfolgt:
bei einer Wellenlänge von 302 nm;
in Küvetten aus optischem Spezialglas: 1 cm Schichtdicke;
bei einer Temperatur von 20 - 25°C,
die Messung wird gegen $H_2O$ durchgeführt.

Es werden in Reagenzgläser pipettiert:

| | Reagentienleerwert | Probenleerwert | Probe |
|---|---|---|---|
| $H_2O$ | 10 µl | --- | --- |
| Probe | --- | 10 µl | 10 µl |
| Reaktionslösung (ohne Nucleosid-phosphorylase | --- | 1000 µl | --- |
| Reaktionslösung | 1000 µl | --- | 1000 µl |

Der Inhalt der Reagenzgläser wird vermischt, es wird 15 Minuten bei 20 bis 25°C inkubiert und die Extinktion gegen $H_2O$ gemessen. Die Extinktion (E) bleibt mindestens 60 Minuten stabil.

Die Berechnung wird wie folgt durchgeführt:

$$\text{Phosphat-Konzentration} = (E_{Probe} - E_{Probenleerwert} - E_{Reagentienleerwert}) \times 13{,}1 \; [mMol/l]$$

Die ermittelten Werte sind in Fig. 1 im Vergleich zum chemischen Test der Firma Merck wiedergegeben.

Die Berechnung beruht auf den Extinktionskoeffizienten für Harnsäure bei 302 nm von $7{,}7 \times 10^3$ $(Mol/l)^{-1}cm^{-1}$. Sind die eingesetzten Reagentien praktisch phosphatfrei, kann $E_{Probe}$ direkt gegen $E_{Probenleerwert}$ bestimmt werden und das Mitführen eines Reagentienleerwertes erübrigt sich. Da Phosphat stöchiometrisch und quantitativ in Harnsäure überführt wird, ist das Mitführen eines Standardwertes nicht erforderlich.

## Beispiel 2

NP-XOD-POD-Farbtest (Methode 1.4.2.)

Die Bestimmung erfolgte nach folgendem Schema:

$$\text{Phosphat} + \text{Inosin} \xrightarrow[\text{(NP)}]{\text{Nucleosid-phosphorylase}} \text{Ribose-1-phosphat} + \text{Hypoxanthin}$$

$$\text{Hypoxanthin} + 2O_2 + 2H_2O \xrightarrow[\text{Superoxiddismutase}]{\text{Xanthinoxidase (XOD)}} \text{Harnsäure} + 2\,H_2O_2$$

$$2H_2O_2 + \text{3,5-Dichlor-2-hydroxybenzolsulfonsäure} + \text{4-Aminophenazon} \xrightarrow[\text{(POD)}]{\text{Peroxidase}} \text{N-(4-Antipyryl)-3-chlor-5-sulfonat-p-benzochinonimin}$$

Die Konzentration des gebildeten substituierten Benzochinonimins wurde bei 546 nm fotometrisch bestimmt.

Es wird folgende Reaktionslösung hergestellt:

| | |
|---|---|
| KCl/Tris-HCL-Puffer, pH 7,4 | 104 mM/50 mM |
| Ethylendiamintetraacetat | 1 mM |
| Inosin | 0,2 mM |
| 3,5-Dichlor-2-hydroxybenzolsulfonsäure | 4,2 mM |
| 4-Aminophenazon | 0,3 mM |
| Nucleosid-phosphorylase | 500 U/l |
| Xanthinoxidase | 20 U/l |
| Peroxidase | 125 U/l |
| Superoxiddismutase | 20 mg/l |

Der Standard (1,5 mMol/l) wird wie folgt bereitet:

204 mg Kalium-dihydrogenphosphat mit ca. 800 ml bidestilliertem Wasser lösen, 50 ml Schwefelsäure (0,5 Mol/l) zusetzen und mit bidestilliertem Wasser auf 1000 ml auffüllen.

Die Bestimmung erfolgt:
bei einer Wellenlänge von 546 nm;
in Küvetten mit 1 cm Schichtdicke;
bei einer Temperatur von 20 - 25°C;
die Messung wird gegen Reaktionslösung durchgeführt.

Es werden in Reagenzgläser pipettiert:

| | Probe | Standard |
| --- | --- | --- |
| Probe | 5 /ul | --- |
| Standard | --- | 5 /ul |
| Reaktionslösung | 1000 /ul | 1000 /ul |

Der Inhalt der Reagenzgläser wird vermischt, 15 Min. bei 20 - 25°C inkubiert und die Extinktion (E) gegen Reaktionslösung gemessen. Die Extinktion bleibt mindesten 60 Min. stabil.

Die Berechnung erfolgt nach folgender Gleichung:

$$\text{Phosphat-Konzentration} = E_{Probe} \times \frac{1,5}{E_{Standard}} \ /mMol/l/$$

Die ermittelten Werte sind in Fig. 2 im Vergleich zum anorganisch-chemischen Test der Firma Merck wiedergegeben.

Das Mitführen eines Probenleerwertes ist aufgrund der hohen Verdünnung und des Ablesens im sichtbaren Bereich nicht erforderlich. Das Mitführen eines Standardwertes ist notwendig, da Phosphat mit dieser Reaktionsfolge auch in Gegenwart von Superoxiddismutase nicht quantitativ zu $H_2O_2$ umgesetzt wird.

Beispiel 3

NP-XOD-Farbtest (Methode 2)

Die Bestimmung erfolgte nach folgendem Schema:

Phosphat + Inosin —Nucleosid-phosphorylase (NP)→ Ribose-1-phosphat + Hypoxanthin

Hypoxanthin + 2 Tetrazoliumsalz —Xanthinoxidase (XOD)→ Harnsäure + 2 Formazan

Als Tetrazoliumsalz wurde INT (2-(p-Jodophenyl)-3-(p-nitro-phenyl)-5-phenyl-tetrazoliumchlorid) eingesetzt. Die Konzentration des gebildeten Formazans kann bei 546 nm fotometrisch bestimmt werden.

Es wird die folgende Reaktionslösung hergestellt:

| | |
|---|---|
| KCl/Tris-HCl-Puffer, pH 7,4 | 104 mM/50mM |
| Ethylendiamintetraacetat | 1mM |
| Triton X-100 | 0,05 % (w/v) |
| Inosin | 0,2 mM |
| INT | 1 mM |
| Nucleosid-phosphorylase | 500 U/l |
| Xanthinoxidase | 20 U/l |

Die Bestimmung erfolgt:
bei einer Wellenlänge von 546 nm;
in Küvetten mit 1 cm Schichtdicke
bei einer Temperatur von 20 - 25°C;
die Messung wird gegen Reaktionslösung durchgeführt.

Es werden in Reagenzgläser pipettiert:

| | |
|---|---|
| Probe | 5 µl |
| Reaktionslösung | 1000 µl |

Der Inhalt der Reagenzgläser wird vermischt, 15 Min. bei 20 - 25°C inkubiert und die Extinktion gegen Reaktionslösung gemessen. Die Extinktion (E) bleibt mindesten 60 Min. stabil.

Die Berechnung erfolgt nach der Gleichung:

Phosphat-Konzentration = $E_{Probe}$ x 8,08 [mMol/l].

Die ermittelten Werte sind in Fig. 3 im Vergleich zum anorganisch-chemischen Test der Frima Merck wiedergegeben.

Die Berechnung beruht auf einem Extinktionskoeffizienten für das gebildete Formazan bei 546 nm von $12{,}4 \times 10^3$ $(Mol/l)^{-1}cm^{-1}$, wobei berücksichtigt wurde, dass pro Mol Phosphat 2 Mol Formazan entstehen. Das Mitführen eines Probenleerwertes ist aufgrund der hohen Verdünnung und des Ablesens im sichtbaren Bereich nicht erforderlich. Das Mitführen eines Standardwertes ist ebenfalls nicht erforderlich, da Phosphat stöchiometrisch und quantitativ zur Bildung des Formazans führt.

Wie ein Vergleich der erfindungsgemässen enzymatischen Methoden mit der Nachweismethode nach Merck zeigt (Fig. 1-3), stimmen die mit der enzymatischen Methode bei allen drei Reaktionsabläufen erhaltenen Werte gut mit den mit der anorganisch-chemischen Methode erzielten Werten überein. Eine Störung durch Arzneimittel konnte bei diesen zufällig gewählten und nicht ausgesuchten Seren nicht festgestellt werden.

# P a t e n t a n s p r ü c h e

1. Verfahren zur enzymatischen Bestimmung von anorganischem Phosphat, dadurch g e k e n n z e i c h n e t , dass anorganisches Phosphat in Gegenwart eines Nucleosids und biokatalytischer Mengen Nucleosid-phosphorylase umgesetzt und entweder die gebildete Base oder Ribose-1-phosphat quantitativ bestimmt wird.

2. Verfahren gemäss Anspruch 1, dadurch g e k e n n z e i c h n e t , dass anorganisches Phosphat in Gegenwart von Inosin und biokatalytisch wirksamen Mengen Nucleosid-phosphorylase zu Hypoxanthin und Ribose-1-phosphat umgesetzt und eine der gebildeten Substanzen quantitativ bestimmt wird.

3. Verfahren gemäss Anspruch 1 und 2, dadurch g e k e n n z e i c h n e t , dass das gebildete Ribose-1-phosphat in Gegenwart von Glucose-1,6-diphosphat und biokatalytisch wirksamen Mengen Phosphogluco-mutase zu Ribose-1,5-diphosphat und Glucose-6-phosphat umgesetzt wird, und letzteres in bekannter Weise quantitativ bestimmt wird.

4. Verfahren gemäss Anspruch 3, dadurch g e k e n n z e i c h n e t , dass das gebildete Glucose-6-phosphat in Gegenwart von $NADP^+$ und biokatalytisch wirksamen Mengen Glucose-6-phosphat-dehydrogenase zu 6-Phospho-glucono-lacton und NADPH umgesetzt wird, wobei die Bildung des letzteren spektralfotometrisch zur quantitativen Bestimmung desselben verfolgt wird.

5. Verfahren gemäss Anspruch 1 und 2, dadurch g e k e n n z e i c h n e t , dass das gebildete Hypoxanthin in Gegenwart von Sauerstoff und biokatalytisch wirksamen Mengen Xanthinoxidase zu Harnsäure und $H_2O_2$ bzw. $O_2^-$ umgesetzt wird.

6. Verfahren gemäss Anspruch 5, dadurch g e k e n n z e i c h n e t , dass die gebildete Harnsäure spektralfotometrisch bestimmt wird.

7. Verfahren gemäss Anspruch 5, dadurch g e k e n n z e i c h n e t , dass der $O_2$-Verbrauch mit Hilfe einer Sauerstoffelektrode bestimmt wird.

8. Verfahren gemäss Anspruch 5, dadurch g e k e n n z e i c h n e t , dass das gebildete $O_2^-$ durch Reaktion mit Cytochrom $c^{3+}$ über die Bildung des rot gefärbten Cytochrom $c^{2+}$-Komplexes fotometrisch quantitativ bestimmt wird.

9. Verfahren gemäss Anspruch 5, dadurch g e - k e n n z e i c h n e t , dass das gebildete $O_2^-$ in Gegenwart von $H^+$-Ionen und biokatalytisch wirksamen Mengen Superoxiddismutase zu $H_2O_2$ umgesetzt wird, und letzteres quantitativ bestimmt wird.

10. Verfahren gemäss Anspruch 9, dadurch g e - k e n n z e i c h n e t , dass das gebildete $H_2O_2$ in Gegenwart von Ethanol und $NAD^+$ sowie biokatalytisch wirksamen Mengen Katalase und Aldehyddehydrogenase über NADH katalytisch bestimmt wird.

11. Verfahren gemäss Anspruch 8, dadurch g e - k e n n z e i c h n e t , dass das gebildete $H_2O_2$ durch eine Peroxidase quantitativ bestimmt wird.

12. Verfahren gemäss Anspruch 11, daduch g e - k e n n z e i c h n e t , dass das gebildete $H_2O_2$ in Gegenwart von 4-Aminophenazon und 3,5-Dichlor-benzolsulfonsäure sowie biokatalytisch wirksamer Mengen Peroxidase zu N-(4-Antipyryl)-3-chlor-5-sulfonat-p-benzochinonimin umgesetzt wird, dessen Extinktion fotometrisch quantitativ bestimmt wird.

13. Verfahren gemäss Anspruch 1 und 2, dadurch g e k e n n z e i c h n e t , dass das gebildete Hypoxanthin in Gegenwart biokatalytisch wirksamer Mengen Xanthinoxidase bestimmt wird, und letztere als Dehydrogenase fungiert.

14. Verfahren gemäss Anspruch 13, dadurch g e k e n n z e i c h n e t , dass das gebildete Hypoxanthin in Gegenwart eines Tetrazoliumsalzes und katalytisch wirksamer Mengen Xanthinoxidase zu Harnsäure und Formazan umgesetzt wird, wobei letzteres fotometrisch quantitativ bestimmt wird.

15. Verfahren gemäss Anspruch 14, dadurch g e k e n n z e i c h n e t , dass das verwendete Tetrazoliumsalz INT (2-(p-Jodophenyl)-3-(p-nitrophenyl)-5-phenyl-tetrazoliumchlorid) darstellt.

16. Verfahren gemäss einem oder mehreren der Ansprüche 1 bis 14, dadurch g e k e n n z e i c h n e t , dass anstelle des Nucleosids die entsprechende Desoxyverbindung als Ausgangssubstanz eingesetzt werden kann.

17. Anwendung des Verfahrens gemäss einem oder mehreren der Ansprüche 1 bis 15 zur Bestimmung von anorganischem Phosphat für Routineuntersuchungen in der klinischen Chemie.

18. Anwendung des Verfahrens gemäss einem oder mehreren der Ansprüche 1 bis 15 zur Aktivitätsbestimmung von Phosphat-freisetzenden Enzymen.

19. Anwendung des Verfahrens gemäss einem oder mehreren der Anprüche 1 bis 15 in Kombination mit Phosphatasen zur quantitativen Bestimmung von Phosphoestern.

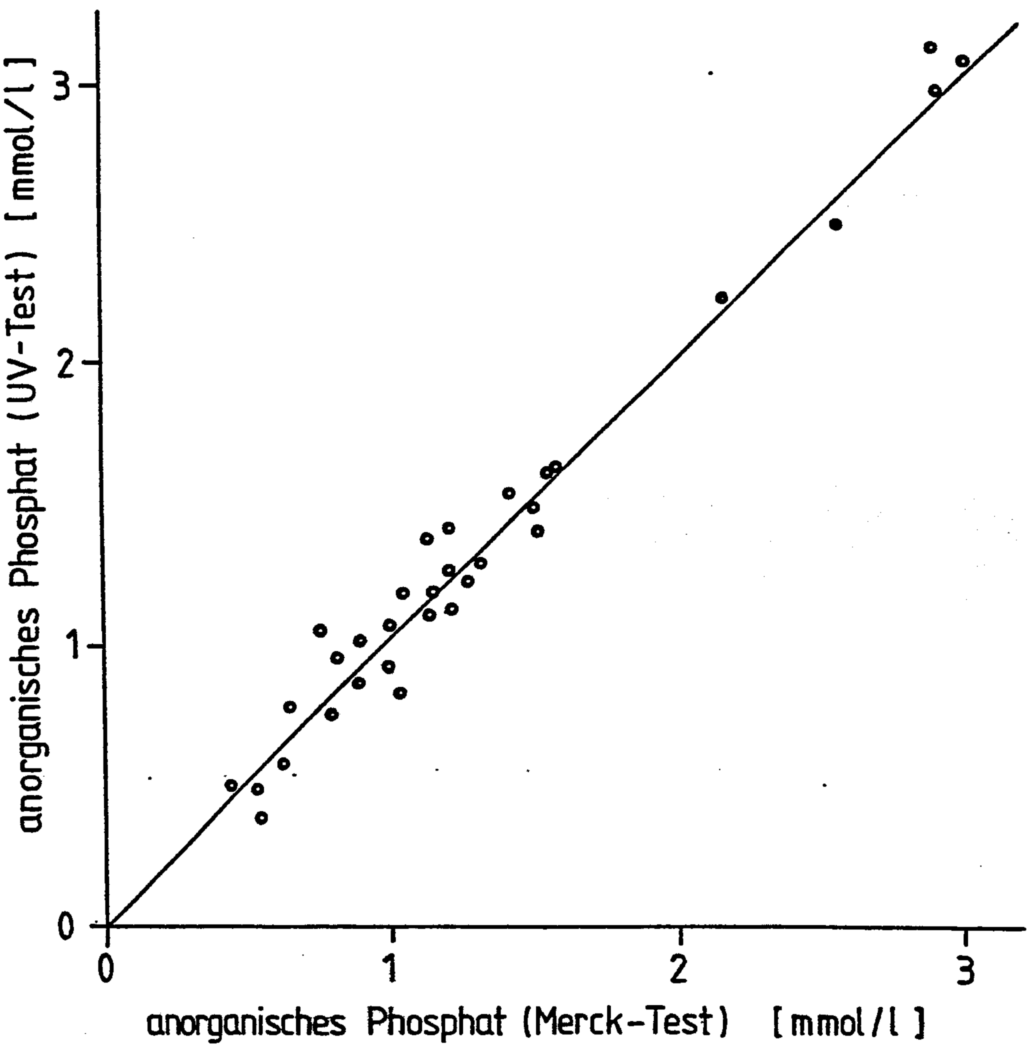

anorganisches Phosphat (UV-Test) [mmol/l]
0
1
2
3
0
1
2
3
anorganisches Phosphat (Merck-Test) [mmol/l]


FIG. 1

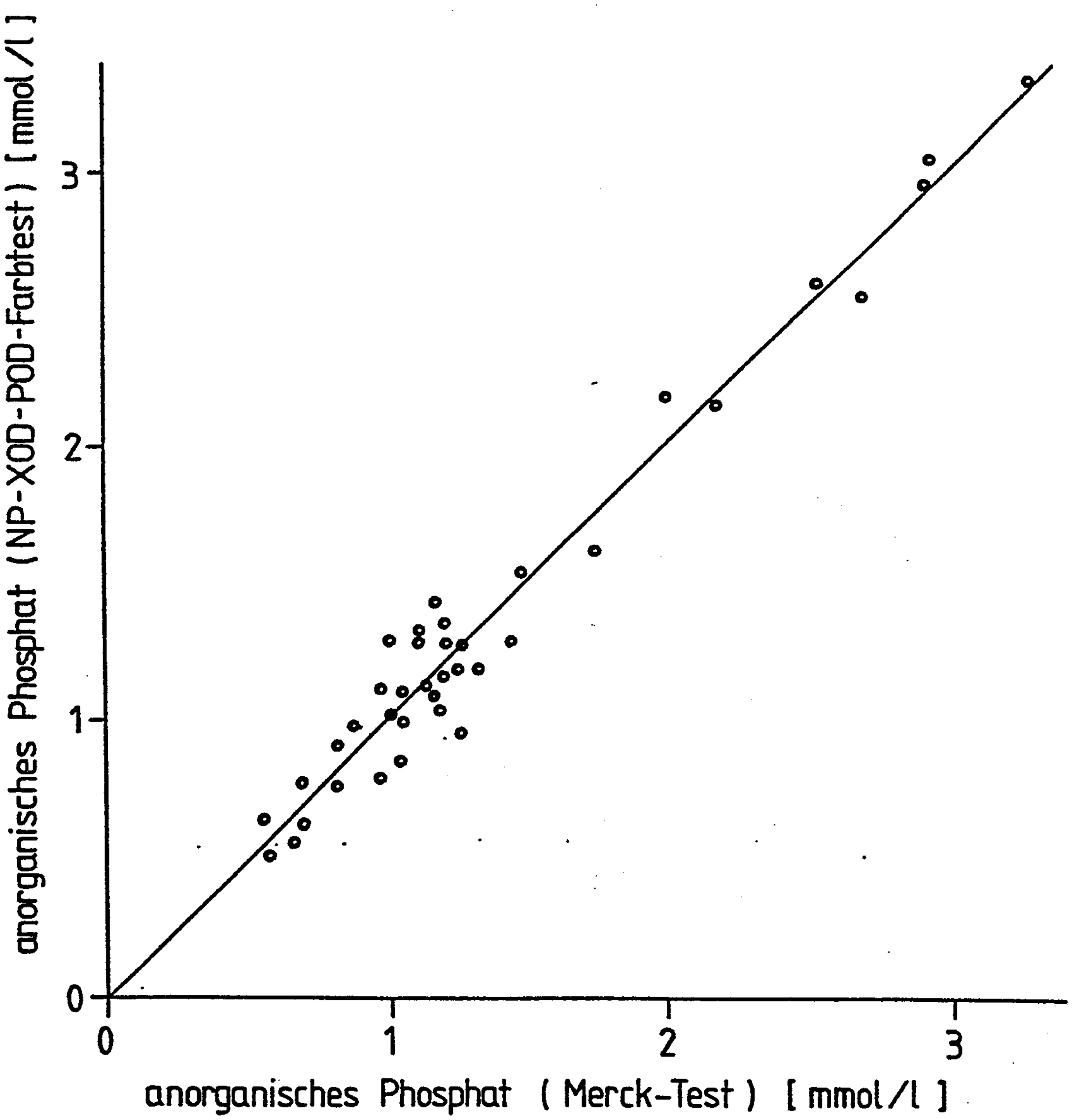
anorganisches Phosphat ( NP-XOD-POD-Farbtest ) [ mmol/l ]
3
2
1
0
0
1
2
3
anorganisches Phosphat ( Merck-Test ) [ mmol/l ]

FIG. 2

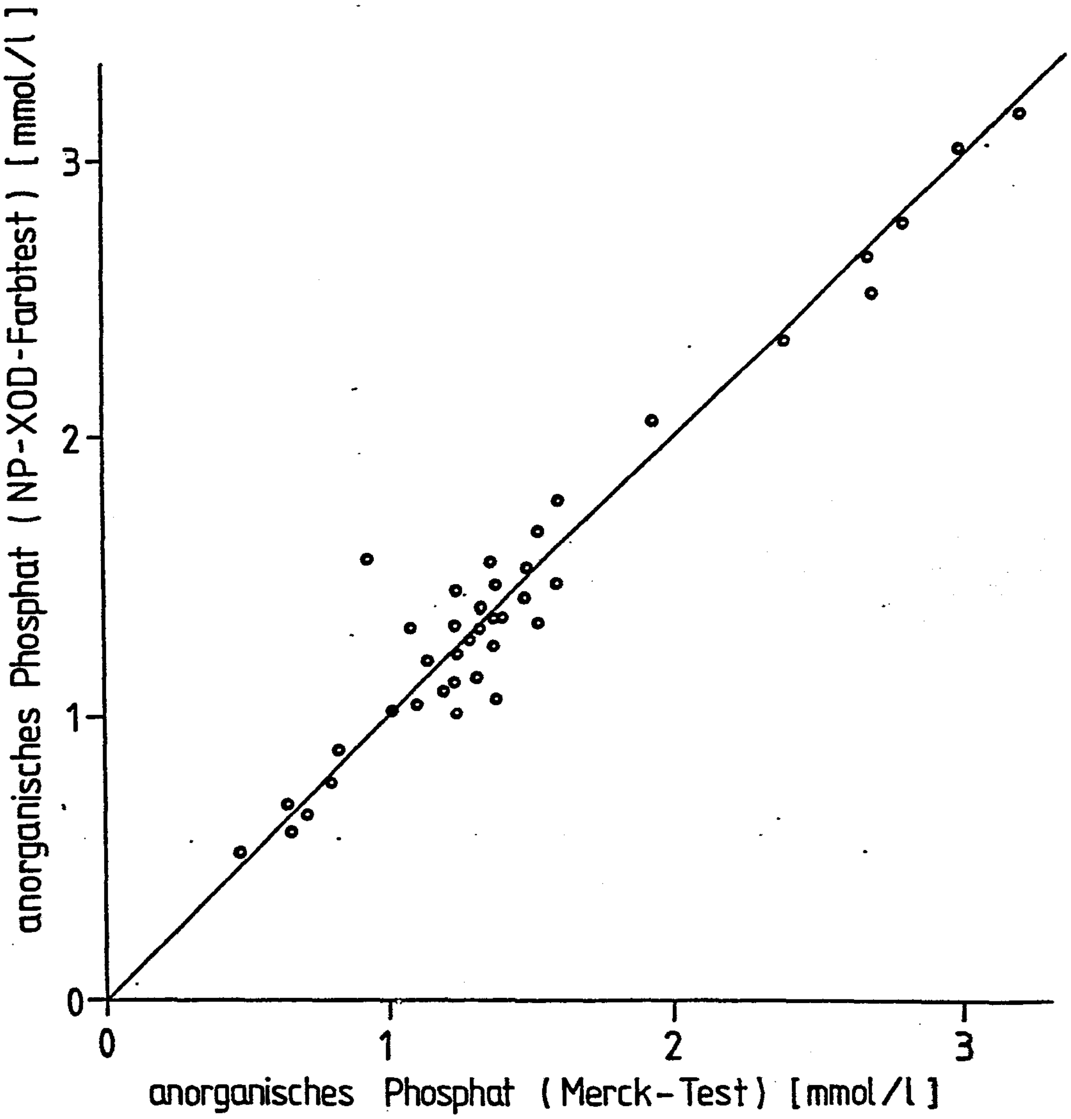
anorganisches Phosphat ( NP-XOD-Farbtest ) [ mmol/l ]
0
1
2
3
0
1
2
3
anorganisches Phosphat ( Merck-Test ) [mmol/l ]

FIG. 3